# EUROPEAN PATENT SPECIFICATION

(11) **EP 3 322 440 B1**
(45) Date of publication and mention of the grant of the patent: **21.08.2019**
(21) Application number: 16748066.4
(22) Date of filing: 13.07.2016
(51) Int. Cl.: A61K 39/085, A61P 17/00

(54) **REDUCING THE RISK OF DEVELOPING OF ATOPIC DERMATITIS BY USE OF SUPERANTIGEN**
VERRINGERUNG DES RISIKOS DER ENTSTEHUNG VON ATOPISCHER DERMATITIS UNTER VERWENDUNG EINES SUPERANTIGENS
RÉDUCTION DU RISQUE DE DÉVELOPPEMENT D'UNE DERMATITE ATOPIQUE PAR UTILISATION D'UN SUPER-ANTIGÈNE

(30) Priority: 14.07.2015 SE 1551023
(43) Date of publication of application: 23.05.2018
(73) Proprietor: Swecure AB, 111 45 Stockholm (SE)
(72) Inventor: WOLD, Agnes, 413 10 Göteborg (SE); ADLERBERTH, Ingegerd, 413 02 Göteborg (SE); HESSELMAR, Bill, 472 94 Svanesund (SE); NOWROUZIAN, Forough, 411 29 Göteborg (SE)
(74) Representative: Ström & Gulliksson AB
(86) International application number: PCT/EP2016/066600
(87) International publication number: WO 2017/009364

(56) References cited:
- WO-A1-2006/009501
- WO-A1-2016/058937
- CN-A- 101 322 842
- M Mempel ET AL: "High Prevalence of Superantigens Associated with the egc Locus in Staphylococcus aureus Isolates from Patients with Atopic Eczema", European Journal of Clinical Microbiology & Infectious Diseases, 1 May 2003 (2003-05-01), pages 306-309, XP055306317, Berlin/Heidelberg DOI: 10.1007/s10096-003-0928-0 Retrieved from the Internet: URL:http://rd.springer.com/content/pdf/10. 1007/s10096-003-0928-0.pdf
- ANNA LÖNNQVIST ET AL: "Neonatal exposure to staphylococcal superantigen improves induction of oral tolerance in a mouse model of airway allergy", EUROPEAN JOURNAL OF IMMUNOLOGY, vol. 39, no. 2, 7 January 2009 (2009-01-07), pages 447-456, XP055100003, ISSN: 0014-2980, DOI: 10.1002/eji.200838418
- John D Fraser ET AL: "The bacterial superantigen and superantigen-like proteins", Immunological reviews, vol. 225 1 October 2008 (2008-10-01), pages 226-243, XP055306250, Denmark DOI: 10.1111/j.1600-065X.2008.00681.x Retrieved from the Internet: URL:10.1111/j.1600-065X.2008.00681.x

## Description

### Field of the invention

The present invention relates to means for preventing, or reducing the risk of developing, atopic dermatitis in human beings.

### Background

Atopic dermatitis, or atopic eczema, is the most common inflammatory disorder in children and poses a significant burden on the quality of life of the population, as well as on health care resources. Currently, 20% of children in affluent countries are affected. Atopic dermatitis is characterized by itching lesions in typical location. The diagnosis is based on clinical criteria, usually Williams' criteria.

### WILLIAMS' CRITERIA

### Major criteria - Patient must have:

- An itchy skin condition (or parental/caregiver report of scratching or rubbing in a child)

### Minor criteria - Patient should have three or more of the following minor criteria:

### Older children (>4 years) /adults:

- History of itchiness in skin creases (e.g., folds of elbows, behind the knees, front of ankles, around the neck)
- Personal history of asthma or allergic rhinitis
- Personal history of general dry skin in the last year
- Visible flexural dermatitis (i.e., in the bends or folds of the skin at the elbow, knees, wrists, etc.)
- Onset under age 2 years

### Children <4 years (Early onset not always diagnostic in children under 4 years of age)

- History of itching of the cheeks
- History of atopic disease in a first-degree relative
- Eczema of cheeks, forehead and outer limbs

Atopic dermatitis is more common in individuals and families with other atopic disorders, such as IgE-mediated food allergy, asthma and hay fever and is distinct from contact dermatitis, psoriasis and seborrheic dermatitis.

Atopic dermatitis is associated with impaired anti-microbial responses in the skin and secondary infections are common in patients with atopic dermatitis.

There is presently no cure for atopic dermatitis. Existing treatments aim at reducing the severity and frequency of flares. Moisturizers and topical corticosteroids are most commonly used to alleviate atopic dermatitis. However, use of corticosteroids is, as known to the skilled person, is associated with side effects, particularly after long-term use.

When topical corticosteroids and moisturizers are not effective in alleviating the symptoms of atopic dermatitis, topical calcineurin inhibitors, such as tacrolimus or pimecrolimus, are sometimes used. However, use of calcineurin inhibitors is a less preferred option as they have been associated with skin cancer or lymphoma. In very severe cases, systemic immunosuppressants, such as ciclosporin, methotrexate, interferon gamma-lb, mycophenolate mofetil and azathioprine, are sometimes used in treating atopic dermatitis.

Given the increasing incidence of atopic dermatitis, it would be desirable to provide means not only for symptomatic treatment, but for counteracting the increasing incidence of atopic dermatitis.

The cause of atopic dermatitis is multifactorial with defects in skin barrier proteins, particularly fillagrin, as an important risk factor. However, genetic factors aside, there is an increasing prevalence world-wide in parallel with improved living standard and better hygiene. Consumption of unpasteurized milk and exposure to dogs during childhood is associated with protection against atopic dermatitis, suggesting that, in common with other atopic diseases and conditions, atopic dermatitis is caused and/or aggravated by a paucity of microbial stimulation in infancy.

Atopic eczematous lesions are often secondarily infected, which worsens the symptoms. *S. aureus* is a common contaminant of eczematous lesions and production of IgE against *Staphylococcus aureus* infecting lesions, or by excreted Staphylococcal enterotoxin B (SEB, a superantigen), which stimulates T-cell responses (cf. WO 2006/104336) is associated with worsening of the symptoms. Accordingly, use of antagonists (e.g. antibodies, antigen-binding protein, and T cell receptor variable regions) to superantigens has been suggested in the art to alleviate atopic dermatitis (cf. WO 2011/028983, WO 2010/030182 and WO 2006/104336).

As present treatment of atopic dermatitis is neither completely successful, nor without side effects, it would be desirable to provide means for preventing, or at least reducing the risk of developing, atopic dermatitis in human beings.

### Summary

The present invention seeks to mitigate, alleviate, circumvent or eliminate at least one, such as one or more, of the above-identified deficiencies. Accordingly there is, according to one aspect of the invention, provided a superantigen selected from the group consisting of the staphylococcal enterotoxins G and I (SEG and SEI) and staphylococcal enterotoxin-like toxins M, N and O (SElM, SElN and SEIO), or a mixture thereof, for use in preventing, or reducing the risk of developing, atopic dermatitis in a human being. In such use the superantigen is to be mucosally administrated to a neonate within 3 months after birth.

According to an aspect of the invention, the superantigen is selected from the group consisting of staphylococcal enterotoxin-like toxins M, N and O (SElM, SElN, and SElO) or from the group consisting of the staphylococcal enterotoxins G and I (SEG and SEI).

According to an aspect of the invention, the superantigen is mucosally administrated to a neonate within 2 weeks after birth, within 10 days after birth, within 7 days after birth, or within 4 days after birth.

According to an aspect of the invention, the superantigen is orally administered, such as via sublingual//swallow administration, or the superantigen is rectally administered.

According to another aspect of the invention, the superantigen is present in a pharmaceutical composition comprising the superantigen and a pharmaceutical acceptable excipient and/or carrier. The pharmaceutical composition may be formulated for rectal, oral, nasal, buccal, or sublingual administration. Especially, the pharmaceutical composition may be formulated for oral, buccal, or sublingual administration, or for rectal administration.

Further advantageous features of the invention are defined in the dependent claims. In addition, advantageous features of the invention are elaborated in embodiments disclosed herein.

### Detailed description

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

Superantigens are a class of molecules that cause activation of T-cells in unspecific manner, by binding to MHC class II molecules on antigen-presenting cells and to the T cell receptor of a large portion of all T cells, resulting in polyclonal T cell activation and massive cytokine release. The large number of activated T-cells generates a massive immune response not being specific to any particular epitope on the superantigen. In human beings with a mature immune system, exposure to superantigens is associated with symptom such as rashes and fever. The symptoms may be severe and even include life-threatening symptoms, such as shock and multiple organ failure.

As known in the art, superantigens are mainly associated with *Staphylococcus aureus* and *Streptococcus pyogenes,* but also *Streptococcus equi* has been shown to produce superantigens.

Superantigens produced by *Staphylococcus aureus* include the enterotoxins SEA, SEB, SEC1, SEC2, SEC3, SED, SEE, SEG, SEH, SEI, SEJ, SEK, SEL, SEM, SEN, SEO, SEP, SER, SEQ, SEU, SEV and TSST-1. The superantigens SEK, SEL, SEM, SEN, SEO, SEP, SEQ, and SEU have also been denoted SElK, SElL, SElM, SElN, SELO, SElP, SElQ, and SElU in the art. The letter "l" denotes that they are enterotoxin-like, i.e. that they do have superantigen properties, but that they may have less adverse effects.

In humans, the presence of a complex gut microbiota early in life is deemed to be protective against allergy development in general (cf. the hygiene hypothesis). In the prospective IMMUNOFLORA birth cohort, it was observed that early colonization, i.e. within 4 weeks after birth, in the gut by *S. aureus,* but not by other typical cultivable gut bacteria, was associated with protection from development of food allergy at least during the first 18 months of life (cf. WO 2006/009501, and Lundell, A.C., et al., Increased levels of circulating soluble CD14 but not CD83 in infants are associated with early intestinal colonization with Staphylococcus aureus Clin Exp Allergy, 2007. 37(1): p. 62-71).

The usefulness of superantigens in preventing development of allergy has been confirmed in animal models (cf. Lönnqvist A et al., Neonatal exposure to staphylococcal superantigen improves induction of oral tolerance in a mouse model of airway allergy Eur J Immunol, 2009. 39: 447-456). The effect is however only present in newborns, not in adult mammals having a mature immune system. Further, the mature immune system is very reactive to superantigens, which are the causative agent in classical "food poisoning".

In the study underlying WO 2006/009501 and Clin Exp Allergy, 2007. 37(1): p. 62-71, not only food allergy was studied by also atopic dermatitis. In contrast to the development of food allergies, no positive or negative correlation between early *S*. *aureus* colonization and the development of atopic dermatitis were found. Further, another study has reported that *S. aureus* is more frequent in the gut of 6-month-old children who develop atopic dermatitis than in children who remain healthy (Bjorksten, B., E. Sepp, K. Julge, T. Voor, and M. Mikelsaar. 2001. Allergy development and the intestinal microflora during the first year of life. J. Allergy Clin. Immunol. 108:516-520). This finding was however not supported by the study underlying WO 2006/009501 and Clin Exp Allergy, 2007. 37(1): p. 62-71, in which no difference in *S. aureus* colonization between the groups was seen at 1 week or 1 month of age.

The present inventors have surprisingly found that the early colonization, i.e. within 4 weeks after birth, in the gut by some superantigen-producing *S. aureus* provides protection against development of atopic dermatitis, whereas others seemingly predispose for the development of atopic dermatitis. Especially, it was found that early colonization by *S. aureus* producing SEA predispose for the development of atopic dermatitis later on in life. For the majority of *S. aureus* strains, producing superantigens, no significant trend was found.

However, early colonization by *S. aureus* carrying genes being part of the enterotoxin gene cluster *(egc),* encoding for staphylococcal enterotoxins G and I (SEG and SEI) and for the staphylococcal enterotoxin-like toxins M, N and O (SElM, SElN and SEIO), was found to be significantly correlated with lower incidence of atopic dermatitis by 18 months of age.

Seemingly, the interaction between superantigens and the infantile immune system is even more complex than previously revealed. Stimulation of the infantile immune system by superantigens seems to either predisposes or protects from later development of atopic dermatitis, depending of type of superantigen.

An embodiment of the invention thus relates to a superantigen selected from the group consisting of the staphylococcal enterotoxins G and I (SEG and SEI) and staphylococcal enterotoxin-like toxins M, N and O (SElM, SElN and SElO), or a mixture thereof, for use in preventing, or reducing the risk of developing, atopic dermatitis in a human being. The superantigen is mucosally administrated to provide its effect on the immature immune system of a neonate. As the superantigen is to provide its effect on the infantile immune system, the superantigen is to be administered to the neonate within 3 months after birth. According to an embodiment, atopic dermatitis, as used herein, is in accordance with the Williams' criteria.

### WILLIAMS' CRITERIA

### Major criteria - Patient must have:

- An itchy skin condition (or parental/caregiver report of scratching or rubbing in a child)

### Minor criteria - Patient should have three or more of the following minor criteria:

### Older children (>4 years) /adults:

- History of itchiness in skin creases (e.g., folds of elbows, behind the knees, front of ankles, around the neck)
- Personal history of asthma or allergic rhinitis
- Personal history of general dry skin in the last year
- Visible flexural dermatitis (i.e., in the bends or folds of the skin at the elbow, knees, wrists, etc.)
- Onset under age 2 years

### Children <4 years (Early onset not always diagnostic in children under 4 years of age)

- History of itching of the cheeks
- History of atopic disease in a first-degree relative
- Eczema of cheeks, forehead and outer limbs.

An alternative embodiment relates to the use of a superantigen selected from the group consisting of the staphylococcal enterotoxins G and I (SEG and SEI) and staphylococcal enterotoxin-like toxins M, N and O (SElM, SElN and SEIO), or a mixture thereof, for the manufacture of medicament for use in preventing, or reducing the risk of developing, atopic dermatitis in a human being. The medicament is to be mucosally administrated to the neonate within 3 months after birth. Yet another alternative embodiment relates to a method for preventing, or reducing the risk of developing, atopic dermatitis in a human being, the method comprising mucosally administering the superantigen to a neonate within 3 months after birth. Aspects provided below in relation to a superantigen selected from the group consisting of the staphylococcal enterotoxins G and I (SEG and SEI) and staphylococcal enterotoxin-like toxins M, N and O (SElM, SElN and SEIO), or a mixture thereof, for use in preventing, or reducing the risk of developing, atopic dermatitis in a human being, are equally applicable to these alternative embodiments.

In the study underlying the present invention, the protective effect was slightly more significant for the enterotoxin-like toxins SElM, SElN and SElO. Thus, the superantigen may be selected among these superantigens such as from SElM and SElN. Further, the superantigen may be staphylococcal enterotoxins G or I (SEG and SEI). Further, also any combination of 2, 3, or 4 of SEG, SEI, SElM, SElN, and SEIO, or all of SEG, SEI, SElM, SElN, and SEIO, may be used in preventing, or reducing the risk of developing, atopic dermatitis in a human being.

Further, not only natural superantigens may be used to prevent, or reduce the risk of developing, atopic dermatitis in a human being, but also derivatives thereof, as long as they have superantigen activity. As superantigens are proteins, various ways of obtaining derivatives are known to the skilled person, such as amino acid substitution, deletion, or insertion as well as addition at the N-terminus or C-terminus of the protein. Substitution(s), insertion(s) and addition(s) may be performed with natural as well as non-natural amino acids. One type of derivatives of interest may be fragments of natural superantigens, i.e. proteins and peptides consisting of only part of the sequence of the full-length protein. Further, natural superantigens may be substituted with HIS-tags to facilitate purification, as well as PEG-moieties and other types of moieties affecting the solubility of the protein. According to an embodiment, superantigen, as used herein, relates to natural as well as unnatural superantigens, e.g. derivatives of natural superantigens. According to another embodiment, superantigen, as used herein, relates only to natural superantigens.

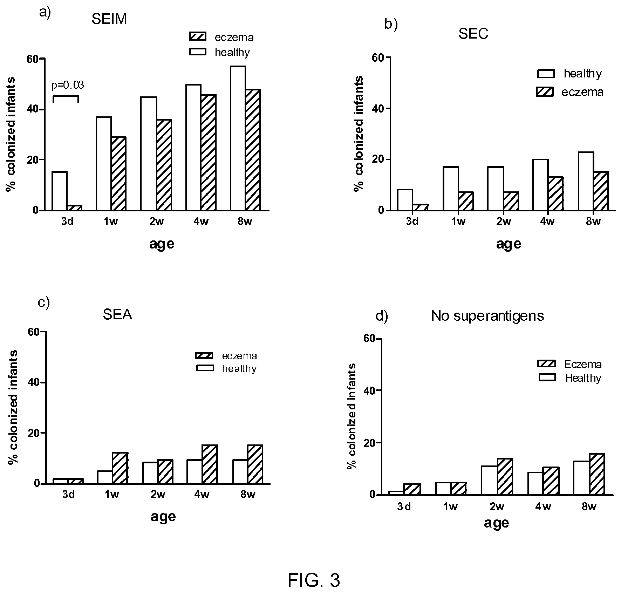

As the superantigen is to exert its effect on the infantile immune system to prevent, or reduce the risk of, development of eczema, it is to be administrated early in life well before the eczematous lesions have appeared. Thus, the superantigen is to be administrated within 3 months after birth. Further, given that the difference in colonization frequency between individuals developing atopic dermatitis and healthy individuals is most pronounced early in life (cf. Fig. 3), it is envisaged that the effect may be more pronounced earlier in life. According to an embodiment, the superantigen is administrated within 2 weeks after birth, within 10 days after birth, within 7 days after birth, or within 4 days after birth. Further, the superantigen may be administred more than once, such as 2, 3, 4, or 5 times.

In order to be able to affect the, e.g. stimulate, the infantile immune system, the superantigen is to be administered mucosally. Various routes for mucosal administration are known in the art. Examples of routes for mucosal administration include the oral, rectal, and nasal route. According to an embodiment, the superantigen is orally administered. In administering the superantigen orally, it may be administered sublingually, buccally, or enterally. According to an embodiment, in which the superantigen is to be administered orally, it is administered via sublingual//swallow administration. According to another embodiment, the superantigen is rectally administered. From a safety perspective, rectal administration may offer some advantages.

According to an alternative, less preferred embodiment, the superantigen is nasally administered.

Typically, the superantigen, being the pharmaceutical active component, is formulated into a pharmaceutical composition in order to facilitate its administration. Apart from the superantigen, the pharmaceutical composition comprises at least one pharmaceutical acceptable excipient and/or carrier. The carrier may also be denoted vehicle.

The pharmaceutical composition may be formulated for rectal, oral, nasal, buccal, or sublingual administration. Preferably, the pharmaceutical composition is formulated for oral, buccal, or sublingual administration.

The pharmaceutical compositions comprising the superantigen may, for example, be in the form of tablets, pills sachets, vials, hard or soft capsules, aqueous or oily suspensions, aqueous or oily solutions, emulsions, powders, granules, syrups, elixirs, lozenges, reconstitutable powders, liquid preparations, sprays, creams, salves, jellies, gels, pastes, ointments, liquid aerosols, dry powder formulations, or HFA aerosols. The pharmaceutical composition may be in a form suitable for administration through oral, e.g. enteral, buccal, or sublingual, routes. Further, but less preferred it may be for administration by inhalation or insufflation (e.g. nasal, tracheal, bronchial) routes. According to an embodiment, the composition is for sublingual administration, such as sublingual swallow administration.

Depending upon the subject to be treated as well as the route of administration, the compositions may be administered at varying doses. A suggested dose concentration of administration of a solution or a suspension of bacterial superantigen(s) is 10 to 100 µg/ml, such as about 40 µg/ml. The dose of the superantigen(s) is according to an embodiment in the range 1 to 750 µg per kg bodyweight, such as 10 to 300 µg per kg bodyweight, 20 to 200 µg per kg bodyweight, or 30 to 150 µg per kg bodyweight.

For oral, e.g. enteral, buccal or sublingual, administration, the bacterial superantigen may be combined with various excipients and/or carriers to provide a pharmaceutical composition. Solid pharmaceutical compositions for oral, e.g. enteral buccal, or sublingual, administration, often include:
- binding agents (for example syrups and sugars, acacia, gelatin, sorbitol, tragacanth, polyvinylpyrrolidone, sodium lauryl sulphate, pregelatinized maize starch, hydroxypropyl methylcellulose, lactose, starches, modified starches, gum acacia, gum tragacanth, guar gum, pectin, wax binders, microcrystalline cellulose, methylcellulose, carboxymethylcellulose, hydroxypropyl methylcellulose, hydroxyethyl cellulose, hydroxypropyl cellulose, copolyvidone and/or sodium alginate);
- disintegrants (such as starch and preferably corn, potato or tapioca starch, alginic acid and certain complex silicates, polyvinylpyrrolidone, sucrose, gelatin, acacia, sodium starch glycollate, microcrystalline cellulose, crosscarmellose sodium, crospovidone, hydroxypropyl methylcellulose and/or hydroxypropyl cellulose),
- lubricating agents (such as magnesium stearate, sodium lauryl sulfate, talc, silica polyethylene glycol waxes, stearic acid, palmitic acid, calcium stearate, carnuba wax, hydrogenated vegetable oils, mineral oils, polyethylene glycols and/or sodium stearyl fumarate); and/or
- fillers (including high molecular weight polyethylene glycols, lactose, sugar, calcium phosphate, sorbitol, glycine magnesium stearate, starch, glucose, lactose, sucrose, rice flour, chalk, gelatin, microcrystalline cellulose, calcium sulphate, xylitol and/or lactitol).

Further, such solid compositions may also include preservative agents and/or anti-oxidants.

Liquid pharmaceutical compositions for oral, e.g. enteral, buccal, or sublingual, administration may be in the form of, for example, solutions, dispersions, emulsions, syrups, or elixirs, or may be presented as a dry product for reconstitution with water or other suitable vehicle before use. Such liquid compositions may contain conventional additives, such as suspending agents (e.g. sorbitol, syrup, methyl cellulose, hydrogenated edible fats, gelatin, hydroxyalkylcelluloses, carboxymethylcellulose, aluminium stearate gel, hydrogenated edible fats), emulsifying agents (e.g. lecithin, sorbitan monooleate, or acacia), aqueous or non-aqueous vehicles (including edible oils, e.g. almond oil, fractionated coconut oil), oily esters (for example esters of glycerine, propylene glycol, polyethylene glycol or ethyl alcohol), glycerine, water or normal saline, preservatives (e.g. methyl or propyl p-hydroxybenzoate or sorbic acid) and conventional flavoring, preservative, sweetening or colouring agents. Diluents such as water, ethanol, propylene glycol, glycerin and combinations thereof may also be included.

Suitable fillers, binders, disintegrants, lubricants and additional excipients are well known to a person skilled in the art.

Formulation factors that require consideration of design of an oral formulation of a protein or polypeptide, such as superantigen and/or an allergen, include the solution behavior of the protein or polypeptide in aqueous and non-aqueous solvents and the effect of ionic strength, solution pH, and solvent type on the stability and structure of the protein or polypeptide. The effect of temperature during formulation on the stability and structure of the protein or polypeptide must also be considered, as should the overall suitability of the formulation for incorporation into an oral dosage form, and particularly into an oral liquid dosage form, such as a gelatin capsule or syrup.

For nasal administration or administration by inhalation, the superantigen may be delivered in the form of a solution, dry powder or suspension. Administration may take place via a pump spray container that is squeezed or pumped by the administrator or through an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. The bacterial superantigen may also be administered via a dry powder inhaler, either as a finely divided powder in combination with a carrier substance (e.g. a saccharide) or as microspheres. The inhaler, pump spray or aerosol spray may be single or multi dose. The dosage may be controlled through a valve which delivers a measured amount of active compound. Nasal administration, and especially administration by inhalation, represents less preferred routes for administering the superantigen.

Without further elaboration, it is believed that one skilled in the art may, using the preceding description, utilize the present invention to its fullest extent. The above preferred specific embodiments are, therefore, to be construed as merely illustrative and not limitative to the disclosure in any way whatsoever.

Although the present invention has been described above with reference to (a) specific embodiment(s), it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc do not preclude a plurality.

### EXPERIMENTAL

### BRIEF DESCRIPTION OF DRAWINGS

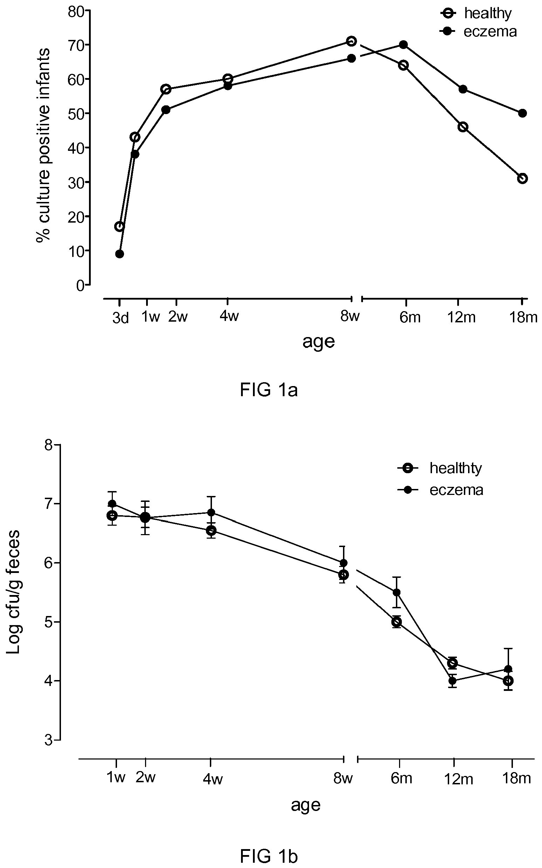

These and other aspects, features and advantages of which the invention is capable of will be apparent and elucidated from the following description of representative embodiments of the present invention, reference being made to the accompanying drawings, in which
**Fig. 1a** depicts intestinal *S. aureus* colonization rate in infants (n = 43) who developed atopic dermatitis and infants (n = 130) who remained healthy during the first 18 months of age;
**Fig. 1b** depicts the number of viable *S. aureus* cells per gram faeces in infants colonized in the gut by *S. aureus.* The mean and SD have given for each time point;

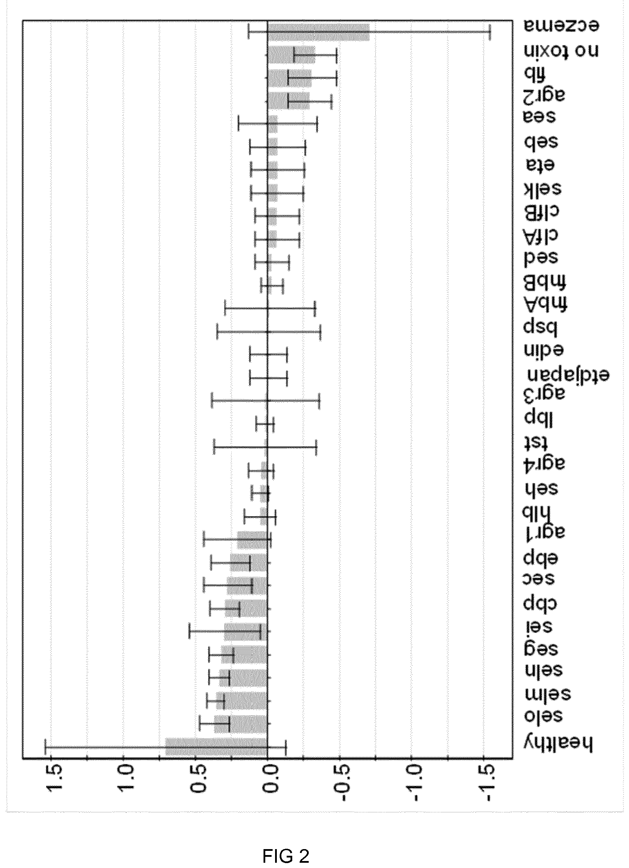

**Fig. 2** depicts orthogonal projection onto latent structures (O-PLS) showing the relation between genes carried by gut colonizing *S. aureus* and the clinical eczematous state at 18 months of age in the child colonized by these bacteria during the two first months of age, i.e. before atopic eczema has appeared. This condition is modeled as Y and the bacterial genes that are positively associated with Y (atopic dermatitis) appear on the right side of the diagram. In contrast, bacterial genes associated to with being healthy at 18 months of age appear on the left. The bar height shows the contribution of the variable and the error bar the unreliability of the contribution.
**Fig. 3** depicts proportion of healthy infants (n = 130) and infants with atopic dermatitis (n = 43) who were colonized with *S. aureus* strains carrying certain bacterial genes (superantigens and adhesins) at different time points during the first two months of life, a) selm-positive *S. aureus,* b) sec-positive *S. aureus,* c) sea-positive, and d) *S. aureus* strains lacking all screened superantigen genes.

### MATERIAL AND METHODS

### Subject

In all 173 Swedish infants born in 1998-2003 at the Sahlgrenska University Hospital, Göteborg, Sweden were included in the ALLERGYFLORA birth-cohort study investigating the relation between infantile intestinal colonization pattern and later allergy development (Adlerberth, I., et al. (2007). "Gut microbiota and development of atopic eczema in 3 European birth cohorts." J Allergy Clin Immunol 120(2): 343-350.). Background factors, such as family, housing conditions, mode of delivery, feeding pattern, illness, medication, etc., were recorded by the parents in a diary and the data are collected during a telephone interview by the study nurse at 6, 12 and 18 months. All parents provided written informed consent for their children to participate in the study. The study was approved by the Ethics Committee of the University of Gothenburg, Sweden (R 448-97 and Ö 446-00).

### Clinical examination

The children were examined clinically at 18 months of age, or when symptoms suggestive of allergy first occurred. Atopic dermatitis was diagnosed according to Williams' criteria (H.C. WILLIAMS et.al. "Diagnostic Criteria for Atopic Dermatitis". British Journal of Dermatology, Volume 131, Issue 3, pages 383-396, September 1994). Food allergy, allergic rhinitis and asthma were diagnosed according to standardized protocols (Hesselmar, B., et al. (2013). "Pacifier cleaning practices and risk of allergy development." Pediatrics 131(6): e1829-1837)..

### Isolation of S. aureus from the infants' intestinal microbiota

A rectal sample was attained at day 3 and cultured aerobically and semi quantitatively. Faecal samples were obtained at 1, 2, 4 and 8 weeks of age and at 6, 12 and 18 months of age and cultured quantitatively for major anaerobic and aerobic bacteria. The methods used for *S. aureus* cultivation, speciation and strain typing have previously been described (Lindberg, E., et al. (2000). "Long-time persistence of superantigen-producing Staphylococcus aureus strains in the intestinal microflora of healthy infants." Pediatr Res 48(6): 741-747, Adlerberth, I., et al. (2007). "Gut microbiota and development of atopic eczema in 3 European birth cohorts." J Allergy Clin Immunol 120(2): 343-350.).). In brief, *S. aureus* strains were isolated after aerobic culture for 2 d on Staphylococus agar. Colonies differing in size, shape, color or transparency were individually enumerated and one colony of each type was speciated based on Gram-staining and the coagulase reaction, thereafter pure-cultured and stored frozen at -80 C°. All *S. aureus* isolates from one infant were analyzed by RAPD (Random amplified polymorphic DNA) for identification of different strains within an infant; isolates from different infants were not compared.

Each strain was analyzed regarding 30 bacterial genes (including superantigens, toxins and adhesins) by a series of multiplex PCR (Nowrouzian, F. L., et al. (2011). "Adhesin and superantigen genes and the capacity of Staphylococcus aureus to colonize the infantile gut." J Infect Dis 204(5): 714-721). The following genes encoding the following virulence factors were analyzed: *S. aureus* enterotoxin (SE) A-D (*sea-d*) and SEH (*seh*); enterotoxin-like (SEl) K (*selk*), SElL (*sell*), SElM (*selm*), SElO (*selo*), SElR (*selr*), SElP (*selp*) and SElQ (*selq*); toxic shock syndrome TSST-1 (tst); exfoliative toxins A, B, and D (*eta, etb,* and *etd*); β-hemolysin (hlb); epidermal cell differentiation inhibitor toxin A-C (*edin*); PVL (*pvl*) and leukotoxin M (l*ukM*); adhesin including fibrinogen-binding protein (*fib*), clumping factors A and B (*clfA* and *clfB*), elastin binding-protein (*ebp*), laminin-binding protein (*lbp*), collagen-binding protein (*cbp*), bone sialoprotein-binding protein (*bsp*), fibronectin-binding proteins A and B (*fnbpA* and *fnbpB*), and the agr allelic variants (*agr 1-4*).

### Statistical analysis

Proportions were compared using Fisher's exact test. Multivariate analysis was performed using the SIMCA-P (Umetrics AB, Umeå, Sweden). Orthogonal projection onto latent structures (O-PLS) was used to reveal the relationship between *S. aureus* virulence genes and the clinical eczematous state at the first 18 months of age. This method is a regression development of principal component analysis (PCA). In O-PLS the clustering data is related to a Y variable, in this case the development of atopic dermatitis.

### RESULTS

A total of 173 infants were followed with regular faecal samples and allergy was diagnosed upon first appearance of symptom and at 18 months of age. The age of debut of symptom was registered.

Twenty five percent of the infants (43/173) developed atopic dermatitis during the first 18 months of age. Of these, 14/43 (33%) also had other allergic manifestations, i.e. food allergy (n = 6), asthma (n = 4), food allergy and asthma (n = 2), food allergy and allergic rhinitis (n = 1) and asthma and allergic rhinitis (n = 1) at 18 months of age.

### Colonization rate and population counts of S. aureus strains

Proportion of children yielding a fecal sample positive for *S. aureus* at different time points during the first 18 months of age is shown in Fig. 1a. Colonization by *S*. *aureus* reached a maximum by 2 months of age in the healthy group, while it increased up to 6 month of age in the eczematous group and thereafter declined in both groups.

The colonization by *S. aureus* in the gut was slightly higher in the healthy group up to 2 months of age (cf. Fig. 1a), but the difference was not significant at any time point:

| | | |
|---|---|---|
| day 3 | 17% vs 9% | (p = 0.2) |
| 1 week | 43% vs 38% | (p = 0.6) |
| 2 weeks | 57% vs 51% | (p = 0.5) |
| 4 weeks | 61% vs 58% | (p = 0.08) |
| 8 weeks | 71% vs 66% | (p = 0.7) |

A decrease in colonization rate after 6 months of age was more pronounced in the healthy group (cf. Fig. 1a), but there was no significant difference in colonization rate between these two groups:

| | | |
|---|---|---|
| 6 months | 64% vs 70% | (p = 0.8) |
| 12 months | 46% vs 57% | (p = 0.2) |
| 18 months | 31% vs 50% | (p = 0.2) |

The counts of *S. aureus* in the faeces obtained from infants were high during the first one month of life thereafter declined sharply (Fig. 1b). This reflects that *S*. *aureus,* which is foremost a skin bacterium is not able to withstand the competition from a more traditional successively more complex microbiota and therefore declines in population numbers.

At no point in time did the *S. aureus* population numbers differ between infants who later developed atopic dermatitis or stayed healthy.

### Gene carriage in intestinal S. aureus strains colonizing healthy infants and infants with eczema prior to début of dermatitis

We examined gene carriage in 138 *S. aureus* strains, i.e. 112 strains obtained from the 93 infants who did not develop atopic dermatitis and 26 strains derived from the 21 infants who developed atopic dermatitis during their first 18 months of life. We chose to study only strains from the first 2 months of age, as we were interested in bacterial factors that would affect the risk of developing eczema and not the effects of eczematous diseases on the microbiota. The few children with already established atopic dermatitis at 2 months of age were excluded from the analysis.

Orthogonal projection onto latent structures (O-PLS) was used to determine the relation between bacterial genes of the intestinal *S. aureus* strains colonizing the infants during the first 2 months of life, and the presence or absence of atopic dermatitis at 18 months of age (the disease might have presented at an earlier age, but later than 2 months of age). Figure 2 shows the bacterial genes in gut *S. aureus* that were associated with healthy state; these factors appear on the left side of diagram. These included the superantigen genes encoded by the *egc* (*seg, sei, selm, seln* and *selo*) as well the *sec* gene and the adhesin genes *cbp* and *ebp.* In contrast, bacterial genes that were associated with having atopic dermatitis at 18 months of age on the right side of the diagram (the larger the bar, the greater the contribution and the smaller error bar, the more reliable the contribution). These include the adhesin gene *fib* and the accessory gene regulator (AGR). Genes contributing little or not at all to the separation are omitted from the diagram.

Carriage rate of different enterotoxin, toxin and adhesin genes among intestinal *S. aureus* strains in relation to the development of atopic dermatitis is also shown in Table 1.

**Table 1 - Prevalence of genes encoding superantigens, other toxins and adhesin S. aureus strains colonizing 0-2 month old infants staying healthy, or developing atopic dermatitis.**

| | **Positive strains %** | | | |
|---|---|---|---|---|
| **Toxin** | **Gene** | **Healthy** n=126 | **Eczema** n=26 | ***P* value** |
| ***Superantigens*** | | | | |
| *Enterotoxin A* | *sea* | 12 | 23 | 0.2 |
| *Enterotoxin C* | *sec* | 29 | 15 | 0.2 |
| *Enterotoxin L* | *sell* | 29 | 15 | 0.2 |
| | | | | |

| *Enterotoxin gene cluster (egc)* | | | | |
|---|---|---|---|---|
| *Enterotoxin G* | *seg* | 73 | 60 | 0.2 |
| *Enterotoxin I* | *sei* | 51 | 36 | 0.2 |
| *Enterotoxin M* | *selm* | 77 | 46 | 0.003 |
| *Enterotoxin N* | *seln* | 80 | 56 | 0.02 |
| *Enterotoxin O* | *selo* | 79 | 62 | 0.07 |
| | | | | |
| *Toxic shock syndrome TSST-1* | *tst* | 22 | 31 | 0.4 |
| *None of superantigens* | - | 16 | 31 | 0.1 |
| | | | | |

| ***Others toxins*** | | | | |
|---|---|---|---|---|
| *Beta-hemolysin* | *hlb* | 24 | 15 | 0.4 |
| *Exfliative toxin A* | *eta* | 3 | 11 | 0.08 |
| | | | | |

| ***Adhesins*** | | | | |
|---|---|---|---|---|
| *Bone sialoprotein binding protein* | *bsp* | 28 | 35 | 0.6 |
| *Collagen binding protein* | *cbp* | 59 | 38 | 0.07 |
| *Elastin binding protein* | *ebp* | 73 | 54 | 0.06 |
| *Fibrinogen binding protein* | *fib* | 64 | 85 | 0.06 |
| *Fibronectin binding protein A* | *fnbA* | 70 | 73 | 0.8 |

The most prevalent superantigen genes were *seg, sei, selm, seln* and *selo,* all encoded by the enterotoxin gene cluster *(egc).* Genes encoding the enterotoxin SElM were more common in strains from subsequently healthy infants in compared to strains from infants later developing atopic dermatitis (p = 0.003). A similar tendency was seen for the seln (p = 0.02) and selo genes (p = 0.07), respectively. In contrast, *S. aureus* strains obtained from infants who developed atopic dermatitis tended more often to carry the *sea* gene encoding the SEA superantigen and the eta gene encoding exofliative toxin in compared with *S. aureus* strains from infants who did not develop atopic dermatitis, although the differences did not reach statistical significance. A lack of all investigated superantigen genes was more common among strains from infants later developing atopic dermatitis than strains from infants staying healthy, but the difference was not significant (Table 1).

These findings show that exposure to especially SElM and SEIO, but also other superantigens encoded by genes in the enterotoxin gene cluster (egc), i.e. staphylococcal enterotoxins G and I (SEG and SEI) and staphylococcal enterotoxin-like toxin N (SElN), early in life provides a protective effect against the development later in life of atopic dermatitis. Thus, it may be concluded that mucosal administration of superantigens SElM and SElO to a neonate may be used to prevent, or reduce the risk of, development of atopic dermatitis in human being. Further, also other superantigens, i.e. staphylococcal enterotoxins G and I (SEG and SEI) and staphylococcal enterotoxin-like toxin N (SElN), encoded by genes in the enterotoxin gene cluster *(egc)* may be useful in preventing, or reducing the risk of developing, atopic dermatitis in a human being.

The agr allele 1 tended to be more common among *S. aureus* strains obtained from infants staying healthy (p = 0.08), while the agr allele 2 was enriched among *S. aureus* strains from infants who developed eczema in the coming period (p = 0.07). The distribution of the agr 3 in these two *S. aureus* collections was roughly similar and few strains belonged to agr 4 (Table 2).

### Bacterial gene carriage in the intestinal S. aureus strains obtained from healthy infants and eczematous infants without other allergies

Fourteen of the 43 infants with atopic dermatitis also developed other allergic manifestations. The strains obtained from these infants were excluded and it was investigated whether the virulence gene profiles still differed between *S. aureus* strains colonizing healthy and subsequently eczematous infants.

**Table 2. Prevalence of genes encoding various toxins and adhesins in S. aureus strains colonizing 0-2 month infants staying healthy or later developing only atopic dermatitis.**

| | **Positive strains %** | | | |
|---|---|---|---|---|
| **Toxin** | **Gene** | **Healthy** n=126 | **Eczema** n=26 | ***P* value** |
| ***Superantigens*** | | | | |
| *Enterotoxin A* | *sea* | 12 | 31 | 0.05 |
| *Enterotoxin C* | *sec* | 29 | 12 | 0.2 |
| *Enterotoxin L* | *sell* | 29 | 15 | 0.2 |
| | | | | |

| *Enterotoxin gene cluster (egc)* | | | | |
|---|---|---|---|---|
| *Enterotoxin G* | *seg* | 73 | 44 | 0.04 |
| *Enterotoxin I* | *sei* | 51 | 12 | 0.006 |
| *Enterotoxin M* | *selm* | 77 | 37 | 0.002 |
| *Enterotoxin N* | *seln* | 80 | 37 | 0.0008 |
| *Enterotoxin O* | *selo* | 79 | 44 | 0.004 |
| | | | | |
| *Toxic shock syndrome TSST-1* | *tst* | 22 | 31 | 0.4 |
| *None of superantigens* | | 16 | 44 | 0.01 |

As shown in Table 2, *S. aureus* strains carrying the superantigen genes that are part of the *egc* (*seg, sei, selm, seln,* and *selo*) were still significantly more common in healthy infants than in infants later developing atopic dermatitis (Table 2). In contrast, the superantigen gene *sea* was more frequent among strains colonizing infants developing eczema compared with those remaining healthy. Strains lacking all screened superantigen genes were significantly enriched in children later developing eczema (Table 2).

The result in Table 2 not only confirmed the trend seen in Table 1, but made it more apparent (i.e. lowered the P-value) for all superantigens encoded by the *egc,* suggesting that these superantigens may be particularly effective in preventing, or reducing the risk for developing eczema, as compared to other atopic manifestations.

Interestingly, the P-value for superantigen gene *sec* remained unchanged (0.2), not confirming that this superantigen was especially effective in preventing development of atopic dermatitis.

### Frequency of infants colonized with S. aureus strains carrying certain virulence factor genes during the first two months of life

Figure 3 shows the proportion of children who were colonized by *S. aureus* strains carrying certain bacterial genes at different time-points; healthy children and infants subsequently developing atopic dermatitis were depicted separately. The analysis confirmed that *S. aureus* carrying genes encoded by the *egc (i.e. selm)* were more commonly found in healthy infants compared with those subsequently developing atopic dermatitis, furthermore the analysis shows that the difference was most pronounced in early sampling time-points (15% vs 2%, p = 0.03 at 3 days of age) (Fig. 3a). Carriage of *S. aureus* strains possessing *sec* gene tended to be more common in infants who stayed healthy (Fig. 3b), while the opposite was true regarding strains carrying *sea,* as well as strains lacking all screened superantigen genes (Fig. 3c-d).

In summary, the results obtained shows that show that exposure to SElM and SEIO, in particular, but also other superantigens encoded by genes in the enterotoxin gene cluster *(egc),* in general, i.e. the staphylococcal enterotoxins G and I (SEG and SEI) and staphylococcal enterotoxin-like toxin N (SElN), early in life provides a protective effect against development later in life of atopic dermatitis.

## Claims

1. A superantigen selected from the group consisting of the staphylococcal enterotoxins G and I (SEG and SEI) and staphylococcal enterotoxin-like toxins M, N and O (SElM, SElN and SEIO), or a mixture thereof, for use in preventing, or reducing the risk of developing, atopic dermatitis in a human being, wherein the superantigen is mucosally administrated to a neonate within 3 months after birth.

2. A superantigen for use according to claim 1, wherein the superantigen is selected from the group consisting of staphylococcal enterotoxin-like toxins M, N and O (SElM, SElN, and SEIO), such as from staphylococcal enterotoxin-like toxins M and N (SElM and SElN).

3. A superantigen for use according to claim 1, wherein the superantigen is selected from the group consisting of the staphylococcal enterotoxins G and I (SEG and SEI).

4. A superantigen for use according to any one of the claims 1 to 3, wherein the superantigen is mucosally administrated to a neonate within 2 weeks after birth, within 10 days after birth, within 7 days after birth, or within 4 days after birth.

5. A superantigen for use according to any one of the claims 1 to 4, wherein the superantigen is orally administered, such as via sublingual//swallow administration.

6. A superantigen for use according to any one of the claims 1 to 4, wherein the superantigen is rectally administered.

7. A superantigen for use according to any one of the claims 1 to 6, wherein the superantigen to be administered is present in a pharmaceutical composition comprising the superantigen and a pharmaceutical acceptable excipient and/or carrier.

8. A superantigen for use according to claim 7, wherein the pharmaceutical composition is formulated for rectal, oral, nasal, buccal, or sublingual administration.

9. A superantigen for use according to claim 8, wherein the pharmaceutical composition is formulated for oral, buccal, or sublingual administration.

10. A superantigen for use according to claim 8, wherein the pharmaceutical composition is formulated for rectal administration.

11. A superantigen for use according to any one of the claims 1 to 10, wherein the atopic dermatitis is in accordance with the Williams' criteria.

## Patentansprüche

1. Ein Superantigen, ausgewählt aus der Gruppe, bestehend aus den Enterotoxinen G und I (SEG und SEI) aus Staphylokokkus und den enterotoxinähnlichen Toxinen M, N und O (SEIM, SEIN und SEIO) aus Staphylokokkus, oder einer Mischung davon, zur Verwendung bei der Vorbeugung oder Reduzierung des Risikos atopische Dermatitis bei einem Menschen zu entwickeln, wobei das Superantigen einem Neugeborenen innerhalb von 3 Monaten nach der Geburt über die Schleimhaut verabreicht wird.

2. Ein Superantigen für die Verwendung gemäß Anspruch 1, wobei das Superantigen ausgewählt ist aus der Gruppe, bestehend aus den enterotoxinähnlichen Toxinen M, N und O (SEIM, SEIN und SEIO) aus Staphylokokkus, wie etwa von enteroxinähnlichen Toxinen M und N (SEIM und SEIN) aus Staphylokokkus.

3. Ein Superantigen zur Verwendung gemäß Anspruch 1, wobei das Superantigen ausgewählt ist aus der Gruppe, bestehend aus den Entrotoxinen G und I (SEG und SEI) aus Staphylokokkus.

4. Ein Superantigen zur Verwendung gemäß einem der Ansprüche 1-3, wobei das Superantigen einem Neugeborenen innerhalb von 2 Wochen nach der Geburt, innerhalb von 10 Tagen nach der Geburt, innerhalb von 7 Tagen nach der Geburt oder innerhalb von 4 Tagen nach der Geburt über die Schleimhaut verabreicht wird.

5. Ein Superantigen zur Verwendung gemäß einem der Ansprüche 1-4, wobei das Superantigen oral verabreicht wird, wie etwa über eine sublinguale/ eine Schluckverabreichung.

6. Ein Superantigen zur Verwendung gemäß einem der Ansprüche 1-4, wobei das Superantigen rektal verabreicht wird.

7. Ein Superantigen zur Verwendung gemäß einem der Ansprüche 1-6, wobei das Superantigen, das verabreicht werden soll, in einer pharmazeutischen Zusammensetzung vorliegt, die das Superantigen und einen pharmazeutisch annehmbaren Hilfsstoff und/oder Träger umfasst.

8. Ein Superantigen zur Verwendung gemäß Anspruch 7, wobei die pharmazeutische Zusammensetzung für eine rektale, orale, nasale, bukkale oder sublinguale Verabreichung formuliert ist.

9. Ein Superantigen zur Verwendung gemäß Anspruch 8, wobei die pharmazeutische Zusammensetzung für eine orale, bukkale oder sublinguale Verabreichung formuliert ist.

10. Ein Superantigen zur Verwendung gemäß Anspruch 8, wobei die pharmazeutische Zusammensetzung für eine rektale Verabreichung formuliert ist.

11. Ein Superantigen zur Verwendung gemäß einem der Ansprüche 1-10, wobei die atopische Dermatitis in Übereinstimmung mit den Williams'-Kriterien steht.

## Revendications

1. Super-antigène sélectionné dans le groupe constitué des entérotoxines staphylococciques G et I (SEG et SEI) et des toxines de type entérotoxine staphylococcique M, N et 0 (SElM, SEIN et SElO), ou un mélange de celles-ci, pour son utilisation pour prévenir, ou réduire le risque de développer, une dermatite atopique chez un être humain, dans lequel le super-antigène est administré par voie muqueuse à un nouveau-né dans un intervalle de trois mois après la naissance.

2. Super-antigène pour son utilisation selon la revendication 1, dans lequel le super-antigène est sélectionné dans le groupe constitué de toxines de type entérotoxine staphylococcique M, N et 0 (SElM, SEIN et SElO), par exemple de toxines de type entérotoxine staphylococcique M et N (SElM et SEIN).

3. Super-antigène pour son utilisation selon la revendication 1, dans lequel le super-antigène est sélectionné dans le groupe constitué des entérotoxines staphylococciques G et I (SEG et SEI).

4. Super-antigène pour son utilisation selon l'une quelconque des revendications 1 à 3, dans lequel le super-antigène est administré par voie muqueuse à un nouveau-né dans un intervalle de 2 semaines après la naissance, dans un intervalle de 10 jours après la naissance, dans un intervalle de 7 jours après la naissance, ou dans un intervalle de 4 jours après la naissance.

5. Super-antigène pour son utilisation selon l'une quelconque des revendications 1 à 4, dans lequel le super-antigène est administré par voie orale, par exemple par administration sublinguale/par déglutition.

6. Super-antigène pour son utilisation selon l'une quelconque des revendications 1 à 4, dans lequel le super-antigène est administré par voie rectale.

7. Super-antigène pour son utilisation selon l'une quelconque des revendications 1 à 6, dans lequel le super-antigène à administrer est présent dans une composition pharmaceutique comprenant le super-antigène et un excipient et/ou un vecteur pharmaceutiquement acceptables.

8. Super-antigène pour son utilisation selon la revendication 7, dans lequel la composition pharmaceutique est formulée pour une administration rectale, orale, nasale, buccale, ou sublinguale.

9. Super-antigène pour son utilisation selon la revendication 8, dans lequel la composition pharmaceutique est formulée pour une administration orale, buccale, ou sublinguale.

10. Super-antigène pour son utilisation selon la revendication 8, dans lequel la composition pharmaceutique est formulée pour une administration rectale.

11. Super-antigène pour son utilisation selon l'une quelconque des revendications 1 à 10, dans lequel la dermatite atopique répond aux critères de Williams.
